# EUROPÄISCHE PATENTANMELDUNG

(11) **EP 0 719 785 A2**
(43) Veröffentlichungstag der Anmeldung: **03.07.1996**
(21) Anmeldenummer: 95116017.5
(22) Anmeldetag: 11.10.1995
(51) Int. Cl.: C07H 15/04

(54) **Verfahren zur destillativen Abtrennung von Fettalkohol aus fettalkoholischen Alkylpolyglycosid-Lösungen**

(30) Priorität: 03.12.1994 DE 4443086
(71) Anmelder: HÜLS AKTIENGESELLSCHAFT, D-45764 Marl (DE)
(72) Erfinder: Grützke, Jürgen, D-44803 Bochum (DE); Schmidt, Stefan, Dr., D-45721 Haltern (DE)

(57) **Zusammenfassung**

Die Erfindung betrifft ein Verfahren zur destillativen Abtrennung von Fettalkoholen aus fettalkoholischen Alkylpolyglycosid-Lösungen unter Zusatz von fettalkoholischen Suspensionen von Magnesiumoxid oder Magnesiumhydroxid. Die Zusätze unterdrücken unerwünschte Schaumwirkung oder Mitreißen von Alkylpolyglycosid im Fettalkohol.

## Beschreibung

Die Erfindung betrifft ein Verfahren zur destillativen Abtrennung von Fettalkoholen aus fettalkoholischen Alkylpolyglycosid-Lösungen, dadurch gekennzeichnet, daß der fettalkoholischen Alkylpolyglycosid-Lösung eine fettalkoholische Suspension von Magnesiumoxid und/oder Magnesiumhydroxid zugesetzt wird.

Alkylpolyglycoside (APG) sind ungiftige, leicht abbaubare oberflächenaktive Substanzen, die als Wasch- und Reinigungsmittel und als Emulgatoren und Dispergatoren verwendet werden.
Alkylpolyglycoside können einstufig oder zweistufig durch Glycosidierung und Umglycosidierung hergestellt werden. Sie enthalten Alkylgruppen von 8 bis 18 Kohlenstoffatomen, wobei Alkylgruppen mit 12 bis 16 Kohlenstoffatomen vorzugsweise vorhanden sind. Der mittlere Polymerisationsgrad liegt bei 1,05 bis 1,5, vorzugsweise bei 1,2 bis 1,5 und ganz besonders bevorzugt bei 1,2 bis 1,4. Als einsetzbare Kohlenhydrate kommen Monosaccharide, wie Pentosen und Hexosen, Disaccharide, wie Saccharose und Maltose, und Polysaccharide, wie Stärke, in Betracht.

Ein einstufiges Herstellungsverfahren wird u. a. in der Patentanmeldung P 41 01 252.6 beschrieben.
Ein zweistufiges Herstellungsverfahren wird in EP-A-0 306 652 angegeben, wobei zunächst durch Glycosidierung mit n-Butanol ein n-Butylglycosid und daraus durch Umglycosidierung mit einem langkettigen Alkohol das gewünschte langkettige Alkylpolyglycosid hergestellt wird.
Die Reaktion wird normalerweise mit einem großen Überschuß an Alkohol durchgeführt, so daß als Reaktionsprodukt ein Gemisch aus Alkylpolyglycosiden und Alkoholen vorliegt. Diese Alkohole beeinträchtigen die anwendungstechnischen Eigenschaften der Alkylpolyglycoside und müssen daher abgetrennt werden.
Bei dem zweistufigen Herstellungsverfahren verbleiben nach erfolgter Umglycosidierung noch geringe Mengen an kurzkettigen Alkoholen in der Reaktionslösung, vornehmlich C₄-Alkohol, der ebenfalls abgetrennt werden muß. Beide Reaktionen, Glycosidierung und Umglycosidierung, werden durch Säure katalysiert. Nach beendeter Reaktion wird mit Alkali- bzw. Erdalkalihydroxiden oder Alkoholaten neutralisiert. Die dabei zugeführten oder entstehenden sowie die bereits enthaltenen Leichtsieder, vornehmlich Wasser und kurzkettige Alkohole, werden wie der überschüssige Fettalkohol abdestilliert. Da die Siedepunkte der Alkohole, vornehmlich mit 8 oder mehr Kohlenstoffatomen, sehr hochliegen und da sich bereits bei Temperaturen über 150 °C u.a. nicht umgesetzte Kohlenhydratreste unter unerwünschter Dunkelfärbung zersetzen, wird die destillative Abtrennung der Alkohole unter Feinvakuum durchgeführt.
Sowohl bei der zweistufigen als auch bei der einstufigen APG-Synthese wird ein Zwischenprodukt erhalten, das wegen des erforderlichen großen Fettalkoholüberschusses eine Lösung von weniger als 50 % festem APG im verwendeten Fettalkohol-Schnitt darstellt. Bei der weiteren Aufarbeitung muß der Fettalkohol vom neutralisierten APG abgetrennt werden, wozu man sich in der Regel Vakuumdestillationsverfahren bedient, wobei üblicherweise Fallfilm-, Kurzweg- oder Dünnschichtverdampfer oder Kombinationen dieser Verdampfertypen verwendet werden.

In den Verdampferstufen kann es zu starker Schaumentwicklung oder zu Mitreißen von APG im Fettalkohol kommen. Beides ist in hohem Maß unerwünscht, weil es das Ergebnis der Fettalkoholabtrennung verschlechtert.
Außerdem besteht dadurch die Gefahr, daß es zu Feststoff-Ablagerungen durch anbackendes APG an verschiedenen Apparateteilen kommt, was auf Dauer die Funktion der Fettalkoholabtrennung beeinträchtigt und sogar zum Ausfall der gesamten Abtrennstufe durch Verstopfungen führen kann.

Es bestand daher die Aufgabe, eine Verfahrenweise bei der Abdestillation von Fettalkohol aus fettalkoholischen Alkylpolyglycosidlösungen zu finden, die unerwünschte Schaumentwicklung unterdrückt und/oder das Mitreißen von Alkylpolyglycosid in den Fettalkoholbrüden vermeidet.
Es wurde überraschend gefunden, daß durch Zusatz einer Suspension von Magnesiumoxid oder Magnesiumhydroxid in Fettalkohol zur fettalkoholischen APG-Lösung die o.g. Schwierigkeiten deutlich vermindert werden können.

Gegenstand der Erfindung ist daher ein Verfahren zur destillativen Abtrennung von Fettalkoholen aus fettalkoholischen Alkylpolyglycosid-Lösungen, dadurch gekennzeichnet, daß der fettalkoholischen Alkylpolyglycosidlösung eine fettalkoholische Suspension von Magnesiumoxid und/oder Magnesiumhydroxid zugesetzt wird.

Die Suspension aus Magnesiumoxid(hydroxid) und Fettalkohol wird der fettalkoholischen APG-Lösung (mit wäßriger Natronlauge bereits vorneutralisiert) kontinuierlich über eine Pumpe zugeführt. Die APG-Lösung kann auch in teilneutralisiertem oder unneutralisiertem Zustand mit der Suspension versetzt werden. Vor Einleitung in die Verdampferstufen werden beide Flüssigkeitsströme in einem Static-Mischer vermischt. Die Konzentration an den genannten Magnesiumverbindungen beträgt 0,01 bis 3 % bezogen auf Alkylpolyglycosid.
Es ergeben sich insgesamt folgende Vorteile:
- Unterdrückung der Schaumentwicklung in den Verdampfern
- das Mitreißen von APG im Fettalkohol wird reduziert
- in den Flash-Behältern, die üblicherweise den Verdampfern vorgeschaltet sind, tritt weniger Verspritzen ein
- der pH-Wert des Produktes in den Verdampferstufen wird im alkalischen Bereich stabilisiert (Nachsäuerungsgefahr wird verringert)
- durch Zusatz des basisch reagierenden Magnesiumoxids bzw. Magnesiumhydroxids wird für die Neutralisation des sauren fettalkoholischen APG (enthält die Katalysator-Säure) weniger wäßrige Natronlauge benötigt. Dies führt zu einem geringeren Wassergehalt des fettalkoholischen APGs, wodurch sich ein besseres Vakuum in den Verdampferstufen ergibt, was wiederum zu einem höheren Wirkungsgrad der Abtrennstufe führt.

## Patentansprüche

1. Verfahren zur destillativen Abtrennung von Fettalkoholen aus fettalkoholischen Alkylpolyglycosid-Lösungen, dadurch gekennzeichnet, daß der fettalkoholischen Alkylpolyglycosid-Lösung eine fettalkoholische Suspension von Magnesiumoxid und/oder Magnesiumhydroxid zugesetzt wird.

2. Verfahren nach Anspruch 1, dadurch gekennzeichnet, daß die fettalkoholische Suspension in die fettalkoholische Alkylpolyglycosid-Lösung mit Hilfe eines statischen Mischers eingemischt wird.

3. Verfahren nach den Ansprüchen 1 und 2, dadurch gekennzeichnet, daß 0,01 - 3 % Magnesiumverbindungen bezogen auf Alkylpolyglycosid verwendet werden.

4. Verfahren nach den Ansprüchen 1 bis 3, dadurch gekennzeichnet, daß das Alkylpolyglycosid ein Alkylpolyglucosid ist.
